# EUROPEAN PATENT SPECIFICATION

(11) **EP 2 521 722 B1**
(45) Date of publication and mention of the grant of the patent: **06.05.2015**
(21) Application number: 11731990.5
(22) Date of filing: 04.01.2011
(51) Int. Cl.: C07D 413/04, C07C 235/42, C07D 209/46, C07D 307/68, C07D 307/88

(54) **PROCESS FOR MAKING A METABOTROPIC GLUTAMATE RECEPTOR POSITIVE ALLOSTERIC MODULATOR - 874**
VERFAHREN ZUR HERSTELLUNG EINES METABOTROPEN GLUTAMAT-REZEPTOR-POSITIVEN ALLOSTERISCHEN MODULATORS - 874
PROCÉDÉ DE FABRICATION D'UN MODULATEUR ALLOSTÉRIQUE POSITIF DU RÉCEPTEUR MÉTABOTROPIQUE DU GLUTAMATE - 874

(30) Priority: 07.01.2010 US 293012 P
(43) Date of publication of application: 14.11.2012
(73) Proprietor: AstraZeneca AB, 151 85 Södertälje (SE)
(72) Inventor: BOYD, Alistair, Macclesfield Cheshire SK10 2NA (GB); FIELDING, Mark Richard, Macclesfield Cheshire SK10 2NA (GB); FORD, James Gair, Macclesfield Cheshire SK10 2NA (GB); FRODSHAM, Lianne, Macclesfield Cheshire SK10 2NA (GB); GOLDEN, Michael D, Macclesfield Cheshire SK10 2NA (GB); LESLIE, Kevin William, Macclesfield Cheshire SK10 2NA (GB); MCKEEVER-ABBAS, Ben, Macclesfield Cheshire SK10 2NA (GB); TOMLIN, Paula, Macclesfield Cheshire SK10 2NA (GB)
(74) Representative: Curran, Clair
(86) International application number: PCT/SE2011/050002
(87) International publication number: WO 2011/084098

(56) References cited:
- WO-A1-00/35859
- WO-A1-2006/020879
- WO-A1-2008/150232
- WO-A1-2008/150232
- WO-A1-2008/150233
- WO-A1-2008/150233
- US-A1- 2009 111 830
- SAFDAR HAYAT ET AL: "An improved method for the synthesis of [gamma]-lactones using sodium bromate and sodium hydrogen sulfite", TETRAHEDRON LETTERS, vol. 42, no. 9, 1 February 2001 (2001-02-01), pages 1647-1649, XP055058833, ISSN: 0040-4039, DOI: 10.1016/S0040-4039(00)02341-8

## Description

### BACKGROUND

The present invention relates to methods for the preparation of an mGluR2 positive allosteric modulator.

The metabotropic glutamate receptors (mGluR) constitute a family of GTP-binding- protein (G-protein) coupled receptors that are activated by glutamate, and have important roles in synaptic activity in the central nervous system, including neural plasticity, neural development and neurodegeneration.

Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase A₂; increases in arachidonic acid release; and increases or decreases in the activity of voltage- and ligand-gated ion channels (Schoepp et al., 1993, Trends Pharmacol. Sci., 14:13 ; Schoepp, 1994, Neurochem. Int., 24:439; Pin et al., 1995, Neuropharmacology 34:1; Bordi & Ugolini, 1999, Prog. Neurobiol. 59:55).

Eight mGluR subtypes have been identified, which are divided into three groups based upon primary sequence similarity, signal transduction linkages, and pharmacological profile. Group-I includes mGluR1 and mGluR5, which activate phospholipase C and the generation of an intracellular calcium signal. The Group-II (mGluR2 and mGluR3) and Group-III (mGluR4, mGluR6, mGluR7, and mGluR8) mGluRs mediate an inhibition of adenylyl cyclase activity and cyclic AMP levels. For a review, *see* Pin et al., 1999, Eur. J. Pharmacol., 375:277-294.

Activity of mGluR family receptors are implicated in a number of normal processes in the mammalian CNS, and are important targets for compounds for the treatment of a variety of neurological and psychiatric disorders. Activation of mGluRs is required for induction of hippocampal long-term potentiation and cerebellar long-term depression (Bashir et al., 1993, Nature, 363:347 ; Bortolotto et al., 1994, Nature, 368:740 ; Aiba et al., 1994, Cell, 79:365 ; Aiba et al., 1994, Cell, 79:377). A role for mGluR activation in nociception and analgesia also has been demonstrated (Meller et al., 1993, Neuroreport, 4: 879; Bordi & Ugolini, 1999, Brain Res., 871:223). In addition, mGluR activation has been suggested to play a modulatory role in a variety of other normal processes including synaptic transmission, neuronal development, apoptotic neuronal death, synaptic plasticity, spatial learning, olfactory memory, central control of cardiac activity, waking, motor control and control of the vestibulo-ocular reflex (Nakanishi, 1994, Neuron, 13:1031; Pin et al., 1995, Neuropharmacology, supra; Knopfel et al., 1995, J. Med. Chem., 38:1417).

Recent advances in the elucidation of the neurophysiological roles of mGluRs have established these receptors as promising drug targets in the therapy of acute and chronic neurological and psychiatric disorders and chronic and acute pain disorders. Because of the physiological and pathophysiological significance of the mGluRs, there is a need for new drugs and compounds that can modulate mGluR function.

Hayat S. et al discusses an improved method of synthesis of [gamma] lactones using sodium bromate and sodium hydrogen sulfite. WO2008/150233 discusses the preparation of 7-methyl-5-(3-piperazin-1-ylmethyl[1,2,4]oxadiazol-5-yl)-2-(4-trifluoromethoxybenzyl)-2,3-dihydroisoindol-1-one. WO00/35859 discusses the preparation of 5-bromo-1,3-dihydroisobenzofuran-1-one. WO2008/150232 discusses the preparation of 7-methyl-5-(3-piperazin-1-ylmethyl-[1,2,4]oxadiazol-5-yl)-2-(4-trifluoromethoxybenzyl)-2,3-dihydroisoindol-1-one.

### DESCRIPTION OF THE INVENTION

The schemes and processes described herein illustrate the preparation of 7-methyl-5-(3-piperazin-1-ylmethyl-[1,2,4]oxadiazol-5-yl)-2-(4-trifluoromethoxybenzyl)-2,3-dihydroisoindol-1-one, having the structure below:

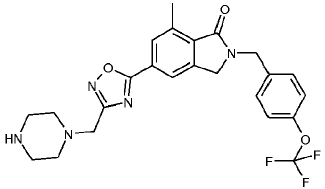

It will be understood by those of skill in the art that the compound made by a process described herein may exist in solvated, for example hydrated, as well as unsolvated forms. It will further be understood that the present invention encompasses all such solvated forms of the compound. Still further, it will be understood by those of skill in the art that the compound may be made by varying, or modifying, the process described herein. Particularly, those of skill in the art will understand that, as examples, solvents, temperatures or specific reagents may be changed or varied and that such changes are within the scope of the present invention.

Within the scope of the invention are also salts of the compound. Generally, pharmaceutically acceptable salts of compound made by the process of the present invention are obtained using standard procedures well known in the art.

In one embodiment of the present invention, the compound may be a pharmaceutically acceptable salt or a solvate thereof. Particularly, a pharmaceutically acceptable salt may be an acidic addition salt such as a hydrochloride, hydrobromide, phosphate, acetate, fumarate, maleate, tartrate, citrate, methanesulphonate or p-toluenesulphonate.

The invention is illustrated by way of the following schemes and examples, which describe several embodiments of the process of the invention. The synthetic schemes and the synthetic procedures provided herein are provided by way of illustration and are not to be construed as limiting the invention. It will be clear to those skilled in the art that the compound or its intermediates may be prepared by modifications of the processes described.

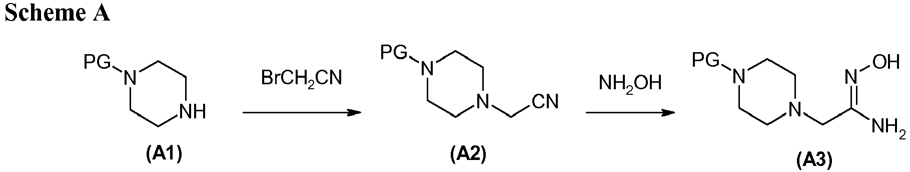

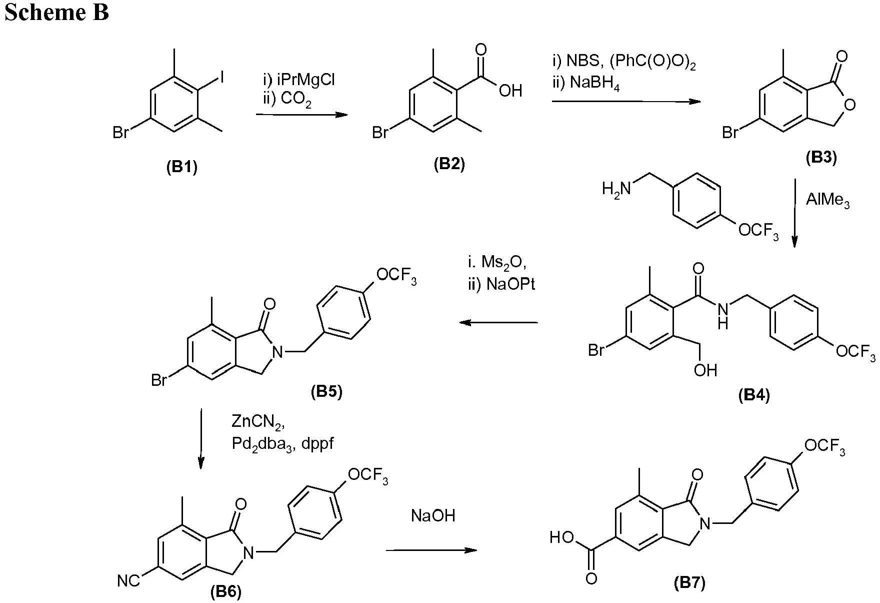

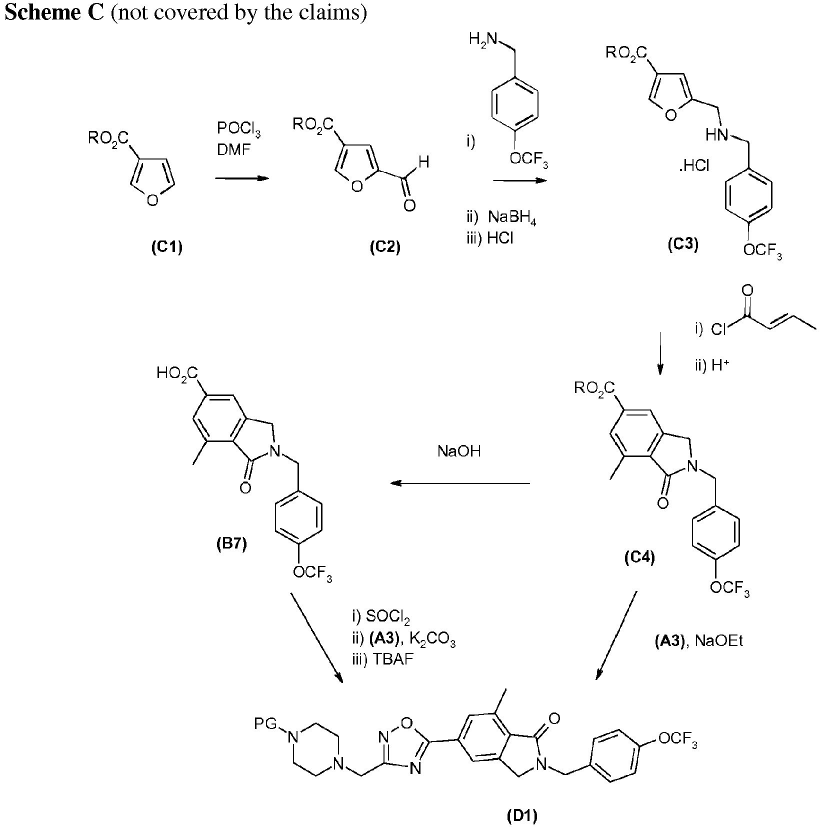

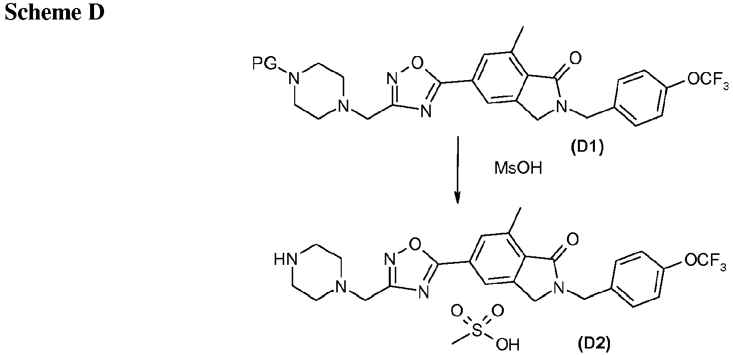

### Definitions:

The following definitions are used in the schemes and descriptions herein:
   NB from schemes, please check and correct where necessary
   "PG" - Protecting Group, for example a BOC group
   "BrCH₂CN" - Bromoacetonitrile
   "NH₂OH" - Hydoxylamine
   "iPrMgCl" - Iso-propylmagnesium chloride
   "NBS" - N-Bromosuccinimide
   "(PhC(CO)O)₂" - Benzoyl peroxide
   "AlMe₃" - Trimethylaluminium
   "Ms₂O" - Methanesulphonyl anhydride
   "NaOPt" - Sodium t-amylate
   "ZnCN₂" - Zinc cyanide
   "R" - A short chain alkyl group, for example, ethyl
   "POCl₃" - Phosphorous oxychloride
   "DMF" - Dimethyl formamide
   "NaBH₄" - Sodium borohydride
   "SOCl₂" - Thionyl Chloride
   "NaOEt" - Sodium ethoxide
   "TBAF" - Tetrabutylammonium fluoride
   "MsOH" - Methane sulfonic acid
   "rel vol" or "rel vols" - Relative volumes

For all the Examples and processes described herein amounts of materials, solvents, volumes, temperatures and times are exemplary and are not to be taken as limiting the manner in which such reactions may be performed or the scope of the invention. Variations in amounts of materials, solvents, volumes, temperatures and times may be made while carrying out the processes described and while practicing the invention described herein.

### Example 1: Preparation of 4-(N-hydroxycarbamimidoylmethyl)-piperazine-1-carboxylic acid tert-butyl ester (A3a)

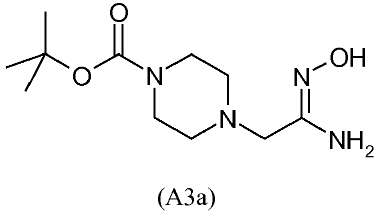

The title compound may be prepared according to Scheme A, as follows. Bromoacetonitrile (1.20 mol eq) may be charged to a cooled solution of tert-butyl piperazine-1-carboxylate (A1a) (1.00 mol eq) and tetramethylguanidine (1.45 mole eq) in tetrahydrofuran (3.6 rel vols). When the reaction is complete the mixture may be warmed, 2-methyltetrahydrofuran (5.0 rel vols) added and then washed with water (1.50 rel vols). The organic solution may be concentrated by vacuum distillation before methanol (1.10 rel vols) is added and the mixture cooled. Hydroxylamine hydrochloride (1.70 mol eq), tetramethylguanidine (1.70 mol eq) and water (0.25 mol eq) may be added, and the mixture heated. When the reaction is complete the mixture may be charged with sodium chloride (0.15 rel wt) and allowed to separate into 2 phases. The lower phase may be removed and back-extracted twice with 2-methyltetrahydrofuran (1.40 rel vols). The combined organic phases may be mixed with heptane (8.0 rel vols), cooled, and seeded. The mixture may be held before cooling further and charging more heptane. The title compound (A3a) may be recovered by filtration, washed once with cold water and twice with heptane and then dried under vacuum (69%).
¹H NMR (400MHz, CDCl3) δ: 1.46 (s, 9H), 2.42 (m, 4H), 3.00 (s, 2H), 3.43 (m, 4H), 5.10 (s, 2H).

### Example 2: Preparation of 4-bromo-2,6-dimethylbenzoic acid (B2)

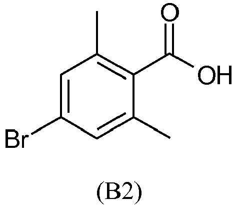

The title compound may be prepared according to Scheme B, as follows. A solution of 5-bromo-2-iodo-1,3-dimethyl-benzene (B1) (1.0 mol eq) in methyl-tert-butyl ether (6.0 rel vols) may be added to iso-propylmagnesium chloride (2.0 mol eq) in methyl-tert-butyl ether (2.0 rel vols), maintaining < 0 °C. Carbon dioxide gas may be added until reaction is shown to be complete. 2N aqueous hydrochloric acid may be added (4 rel vols) to quench the reaction, the phases can be separated and the aqueous phase discarded. The product may be extracted into 1M aqueous sodium hydroxide solution (6.5 rel vols), then washed with methyl tert-butyl ether (4 rel vols). The title compound (B2) may be precipitated by the addition of 2N aq hydrochloric acid (4.5 rel vols) before being filtered, washed with water then heptane and dried to yield a white crystalline solid (85%).
¹H NMR (400 MHz, CDCl₃) δ_{H} 2.28 (6 H, s), 7.35 (2 H, s), 13.30 (1H, s).

### Example 3: Preparation of 5-bromo-7-methyl-3H-isobenzofuran-1-one (B3)

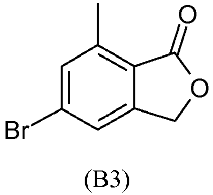

The title compound may be prepared according to Scheme B, as follows. A slurry of 4-bromo-2,6-dimethylbenzoic acid (B2) (1.0 mol eq), N-bromosuccinimide (2.5 mol eq) and benzoyl peroxide (0.1 mol eq) in chlorobenzene (10 rel vols) may be heated to ≥70 °C until the reaction is shown to be complete. A solution of 40% w/w aqueous sodium sulfite (4 rel.vol) may be added to the reaction mixture, the phases separated and the aqueous phase discarded. The organic phase may be washed with saturated aqueous sodium bicarbonate (6 rel vol). The chlorobenzene phase may be concentrated (to 3 rel vols) by vacuum distillation and dimethylacetamide added (2.5 rel vols), before adding this solution to a mixture of sodium borohydride (1.6 mol eq) in dimethylacetamide (3 rel vol). The mixture may be stirred until the reaction is shown to be complete. The mixture may be then quenched with a solution of 36% w/w hydrogen chloride (2.5 mol eq) in water (3.5 rel vol). Removing solvent by vacuum distillation will precipitate the title compound (B3) as a white crystalline solid, which may be filtered and dried (59%).
¹H NMR (400 MHz, DMSO) δ_{H} 2.57 (s, 3H), 5.32 (s, 2H), 7.60 (s, 1H), 7.73 (s, 1H).

### Example 4: Preparation of 4-bromo-2-hydroxymethyl-6-methyl-N-(4-trifluoromethoxy-benzyl)-benzamide (B4)

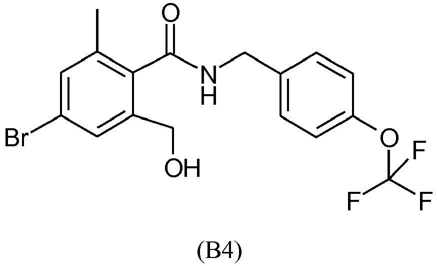

The title compound may be prepared according to Scheme B, as follows. 4-(Trifluoromethoxy)-benzylamine (1.2 mol eq) may be added to a solution of 5-bromo-7-methyl-3H-isobenzofuran-1-one (B3) (1.0 mol eq) in 2-methyltetrahydrofuran (10 rel vols) and inerted. Trimethylaluminium (1.2 mol eq) may be added and the resulting solution heated to ≥40 °C until the reaction is complete. The organic solution may be then added to a cooled solution of potassium sodium tartrate (1.5 mol eq) in water (3 rel vols) and stirred. This biphasic mixture may be separated, and the aqueous phase discarded. The organic phase may be washed with water (3.33 rel vols), then nonane (3 rel vols) may be added and the 2-methyltetrahydrofuran removed by vacuum distillation to crystallise (B4) as a white crystalline solid, which may be filtered, washed with nonane and dried (73.5%).
¹H NMR (400 MHz, DMSO) δ_{H} 2.19 (s, 3H), 4.44 (dd, 4H, 13.4 Hz, 5.78 Hz), 5.33 (t, 1H, 5.76 Hz), 7.34 (s, 1H), 7.35 (s, 2H), 7.47 (s, 1H), 7.49 (s, 2H), 8.85 (t, 1H, 5.76 Hz).

### Example 5: Preparation of 5-bromo-7-methyl-2-(4-trifluoromethoxy-benzyl)-2,3-dihydroisoindol-1-one (B5)

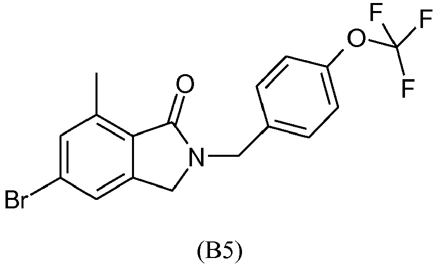

The title compound may be prepared according to Scheme B, as follows. Triethylamine (1.4 mol eq) may be added to 4-bromo-2-hydroxymethyl-6-methyl-N-(4-trifluoromethoxybenzyl)-benzamide (B4) (1.0 mol eq) in 2-methyltetrahydrofuran (5 rel vols). Methanesulphonyl chloride (1.1 mol eq) may be added to this solution, maintaining <5 °C. This may be stirred until the reaction is shown to be complete. Sodium tert-amylate (2.5 mol eq) may be added while maintaining the temperature at <5 °C and the reaction stirred until reaction is completed. Water (10 rel vols) may be added and the resulting biphasic mixture separated, discarding the aqueous phase. 2.7 M aqueous hydrochloric acid (4 rel vols) may be added and the phases separated, discarding the aqueous phase. The organic phase may be concentrated to dryness and the resulting wax recrystallised in heptane (4 rel vols). Filtering, washing with heptane and drying, will afford the title compound (B5) as a white solid (77%).
¹H NMR (400 MHz, DMSO) δ_{H} 2.64 (s, 3H), 4.33 (s, 2H), 4.73 (s, 2H), 7.34 (d, 2H, 8.40Hz), 7.42 (d, 2H, 8.40Hz), 7.47 (s, 1H), 7.59 (s, 1H).

### Example 6: Preparation of 7-methyl-1-oxo-2-(4-trifluoromethoxy-benzyl)-2,3-dihydro-1H-isoindole-5-carbonitrile (B6)

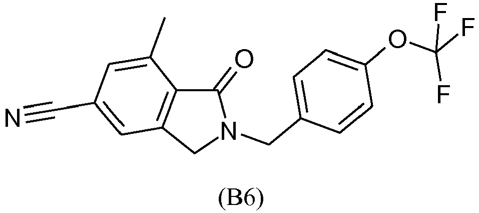

The title compound may be prepared according to Scheme B, as follows. 5-Bromo-7-methyl-2-(4-trifluoromethoxy-benzyl)-2,3-dihydro-isoindol-1-one (B5) (1.0 mol eq), zinc cyanide (0.6 mol eq), 1,1'-bis(diphenylphosphino)ferrocene (0.012 mol eq), tris(dibenzylideneacetone)-dipalladium (0) (0.005 mol eq), dimethylformamide (5 rel vols) and water (0.25 mol eq) can be charged to a vessel and thoroughly inerted with nitrogen before heating to ≥90 °C until the reaction is complete. The reaction may be cooled and t-methyl butyl ether (13 rel vols) added with subsequent filtration. The organic phase obtained may be washed twice with 8.75% w/w aqueous ammonium hydroxide (14 rel vols). Heptane (14 rel vols) may be added to crystallize title compound (B6). Washing with a 9:1 heptane/MTBE mix and drying will afford the title compound (B6) as a brown solid (40%).
¹H NMR (400 MHz, DMSO) δ_{H} 2.69 (d, 3H), 4.41 (s, 2H), 4.76 (s, 2H), 7.34 (s, 1H), 7.36 (s, 1H), 7.42 (s, 1H), 7.44 (s, 1H), 7.74 (s, 1H), 7.86 (s, 1H).

### Example 7: Preparation of 7-methyl-1-oxo-2-(4-trifluoromethoxy-benzyl)-2,3-dihydro-1H-isoindole-5-carboxylic acid (B7)

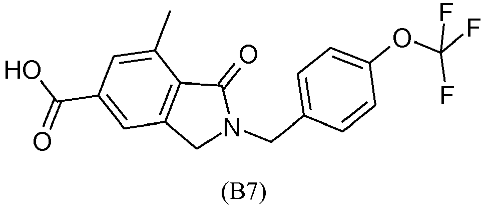

The title compound may be prepared according to Scheme B, as follows. 7-Methyl-1-oxo-2-(4-trifluoromethoxy-benzyl)-2,3-dihydro-1H-isoindole-5-carbonitrile (B6) maybe mixed with methanol (10 rel vols) and 49% aqueous potassium hydroxide (3.0 mol eq) and heated until reaction is complete. The mixture may be concentrated under vacuum to remove the methanol and then diluted with aq. hydrochloric acid to precipitate the product, which may be extracted with iso-propyl acetate. The organic phase may be concentrated to remove solvent, then triturated with heptane, filtered and dried to afford the title compound (B7) (85%).
¹H NMR (400MHz, DMSO) δ: 2.71 (s, 3H), 4.42 (s, 2H), 4.77 (s, 2H), 7.35 (d, 2H, 8.73Hz), 7.43 (d, 2H, 8.73Hz), 7.81 (s, 1H), 7.91 (s, 1H), 13.19 (s, 1H, br).

### Example 8a: Preparation of 5-formyl-furan-3-carboxylic acid ethyl ester (C2a)

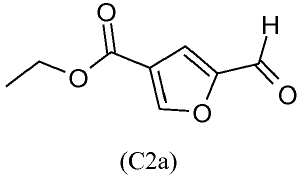

The title compound may be prepared according to Scheme C, as follows. To a solution of ethyl-3-furoate (C1a) (1 mol eq) in N,N-dimethyl formamide (4 mol eq) may be added phosphoryl chloride (4 mol eq). The resulting solution may be heated to 60 °C until reaction is deemed complete. The reaction mixture may be then added to a solution of sodium carbonate (8.25 mol eq) in water (18 rel vols). Toluene (7 rel vols) may be added, and the phases separated. The organic phase may be concentrated (to 4.2 rel vols total volume) under atmospheric pressure. This will afford a 22.1% w/w solution of the title compound (C2a) as 3.5% w/w solution in toluene (73%).
¹H NMR (400 MHz, CDCl₃) δ_{H} 1.37 (3 H, t, 7.2Hz), 4.33 (2 H, q, 7.2Hz), 7.54 (1 H, m), 8.22 (1 H, m) and 9.69 (1 H, m).

### Example 8b: Preparation of 5-formyl-furan-3-carboxylic acid ethyl ester (C2a)

The title compound may be prepared according to Scheme C by use of a flow reactor (for example, a 2-train Continuously Stirred Tank Reactor (CSTR)). A CSTR may be fed with ethyl-3-furoate (C1a) (1 mol eq), N,N-dimethyl formamide (3.833 mol eq) and phosphoryl chloride (4.167 mol eq). The reaction temperature may be maintained at 105 °C, and the feed rates adjusted to yield a mean residence time of 2 hours. The solution leaving the reactor may be quenched into a solution of potassium bicarbonate (8.33 mol eq) in water (3.13 L / g potassium carbonate). Toluene (0.26 L / L water) may be added, and the phases separated. The organic phase may be concentrated (by 3.2 volumes) via distillation under atmospheric pressure. This will afford a 22.4% w/w solution of the title compound (C2a) in toluene.
¹H NMR (400 MHz, CDCl₃) δ_{H} 1.37 (3 H, t, 7.2Hz), 4.33 (2 H, q, 7.2Hz), 7.54 (1 H, m), 8.22 (1 H, m) and 9.69 (1 H, m).

### Example 9: Preparation of 5-[(4-trifluoromethoxy-benzylamino)-methyl]-furan-3-carboxylic acid ethyl ester hydrochloride (C3a)

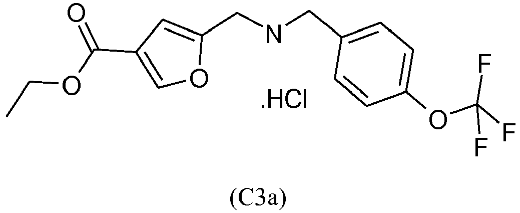

The title compound may be prepared according to Scheme C, as follows. To a 22.1%w/w solution of 5-formyl-furan-3-carboxylic acid ethyl ester (C2a) (1 mol eq) in toluene, 4-(trifluoromethoxy)-benzylamine (1.1 mol eq) may be added, and the reaction stirred until the reaction is complete. Sodium borohydride (1.25 mol eq) may be added and the resulting slurry cooled to ≥-5 °C. Ethanol (16.0 rel.vol.) may be added and the resulting solution quenched with aqueous hydrochloric acid (3.0 mol eq) to crystallise the title compound (C3a). This slurry may be heated to ≥60 °C and then cooled to improve the crystal form. Filtering and washing with toluene and then aqueous ethanol will afford the title compound (C3a) as a crystalline white solid (85%).
¹H NMR (400MHz, DMSO) δ_{H} 1.28 (3 H, t), 4.24 (6 H, m), 6.97 (1 H, d), 7.44 (1 H, d), 7.70 (1 H, d), 8.46 (1 H, d).

### Example 10: Preparation of 7-methyl-1-oxo-2-(4-trifluoromethoxy-benzyl)-2,3-dihydro-1H-isoindole-5-carboxylic acid ethyl ester (C4a)

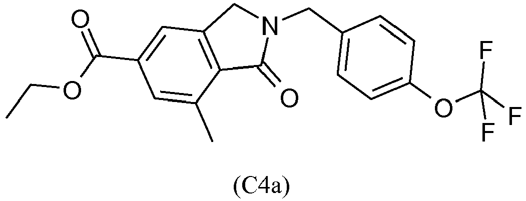

The title compound may be prepared according to Scheme C, as follows. Crotonoyl chloride (1.05 mol eq) may be added to a mixture of 5-[(4-trifluoromethoxy-benzylamino)-methyl]-furan-3-carboxylic acid ethyl ester hydrochloride (C3a) (1.0 mol eq), diisopropylethylamine (2.5 mol eq) and toluene (10 rel vols) at ambient temperature. When the reaction is complete, the mixture may be washed with water (5 rel vols), concentrated (to 5 rel vols total volume) and heated to reflux for > 12 hours. Methanesulphonic acid (1.0 mol eq) may be added, and the reaction then heated to reflux under Dean & Stark conditions until reaction is deemed complete. The solution may be then washed with aqueous sodium hydroxide solution (5 rel vols) then water (5 rel vols). Heptane (10 rel vols) may be added, and the mixture cooled to permit crystallization to occur. The mixture may be filtered and the recovered solid dried to afford the title compound (C4a) as a white crystalline solid (73%).
¹H NMR (400MHz, CDCl3) δ: 1.40 (t, 3H, 7.12Hz), 2.81 (s, 3H), 4.27 (s, 2H), 4.39 (q, 2H, 7.12Hz), 4.78 (s, 2H), 7.18 (d, 2H, 8.60Hz), 7.35 (d, 2H, 8.19Hz), 7.87 (s, 1H), 7.90 (s, 1H).

### Example 11: Preparation of 7-methyl-1-oxo-4-[4-(trifluoromethoxy)-benzyl]2-,3-dihydro-1H-isoindole-5-carboxylic acid (B7)

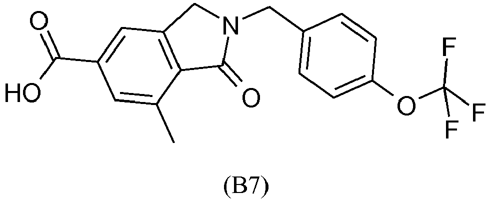

The title compound may be prepared as according to Scheme C, as follows. 7-Methyl-1-oxo-2-(4-trifluoromethoxy-benzyl)-2,3-dihydro-1H-isoindole-5-carboxylic acid ethyl ester (C4a) may be mixed with 2-propanol (4 rel vols) and 49% aqueous potassium hydroxide (1.5 mol eq) and heated until reaction is complete. 36% w/w aqueous hydrochloric acid (2.00 mol eq), may be added, the reaction mixture cooled to ambient temperature and water (5 rel vols) added. The title compound (B7) may be recovered by filtration and dried (98%).
¹H NMR (400MHz, DMSO) δ: 2.71 (s, 3H), 4.42 (s, 2H), 4.77 (s, 2H), 7.35 (d, 2H, 8.73Hz), 7.43 (d, 2H, 8.73Hz), 7.81 (s, 1H), 7.91 (s, 1H), 13.19 (s, 1H, br).

### Example 12a: Preparation of 4-{5-[7-methyl-1-oxo-2-(4-trifluoromethoxy-benzyl)-2,3-dihydro-1H-isoindol-5-yl]-[1,2,4]oxadiazol-3-ylmethyl}-piperazine-1-carboxylic acid tert-butyl esther (D1a)

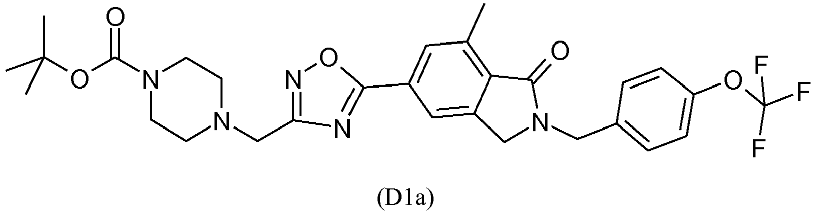

The title compound may be prepared according to Scheme C, as follows. 4-(N-Hydroxycarbamimidoylmethyl)-piperazine-1-carboxylic acid tert-butyl ester (A3a) (1.0 mol eq) and 7-methyl-1-oxo-2-(4-trifluoromethoxy-benzyl)-2,3-dihydro-1H-isoindole-5-carboxylic acid ethyl ester (C4a) (1.0 mol eq) can be mixed in acetonitrile (14 rel vols) at 60 °C. Sodium ethoxide (0.2 mols) may be added over 20 mins and the reaction held at 60 °C until complete. Water (10 rel vols) may be added and the mixture cooled to ambient temperature. The title compound (D1a) may be recovered by filtration and washed with water before being dried (84%).
¹H NMR (400MHz, CDCl₃) δ: 1.46 (9 H, s), 2.58 (4 H, m), 2.84 (3 H, s), 3.50 (4 H, m), 3.79 (2 H, s), 4.33 (2 H, s), 4.81 (2 H, s), 7.20 (2 H, m), 7.36 (2 H, m), 7.99 (1 H, s), 8.04 (1 H, s).

### Example 12b: Preparation of 4-{5-[7-methyl-1-oxo-2-(4-trifluoromethoxy-benzyl)-2,3-dihydro-1H-isoindol-5-yl]-[1,2,4]oxadiazol-3-ylmethyl}-piperazine-1-carboxylic acid tert-butyl ester (D1a)

The title compound may be prepared according to Scheme C, as follows. A slurry of 7-methyl-1-oxo-2-[4-(trifluoromethoxy)-benzyl]-2,3-dihydro-1H-isoindole-5-carboxylic acid (B7) (1.00 mol eq) in toluene (8.0 rel vols) may be heated and thionyl chloride (1.50 mol eq) added. When the reaction is complete, excess thionyl chloride and toluene can be removed by atmospheric distillation. This solution may be added to a slurry of 4-(N-hydroxycarbamimidoylmethyl)-piperazine-1-carboxylic acid tert-butyl ester (A3 a) (1.1 mol eq) and potassium carbonate (1.25 mol eq) in 2-methyl tetrahydrofuran (19.0 rel vols). Tetrabutylammonium fluoride (1.0 mol eq) in tetrahydrofuran (0.5 rel vols) may be charged and the contents heated until the cyclization is complete. After cooling the organic phase may be washed with water before being concentrated by distillation. Methyl-t-butyl ether may be added to induce crystallization. After cooling, the title compound (D1a) may be recovered by filtration, washed with methyl-t-butyl ether and dried to constant weight in a vacuum oven.
¹H NMR (400MHz, CDCl₃) δ: 1.46 (9 H, s), 2.58 (4 H, m), 2.84 (3 H, s), 3.50 (4 H, m), 3.79 (2 H, s), 4.33 (2 H, s), 4.81 (2 H, s), 7.20 (2 H, m), 7.36 (2 H, m), 7.99 (1 H, s), 8.04 (1 H, s).

### Example 13: Preparation of 7-methyl-5-(3-piperazin-1-ylmethyl-[1,2,4]oxadiazol-5-yl)-2-(4-trifluoromethoxy-benzyl)-2,3-dihydro-isoindol-1-one as a methanesulphonate (D2)

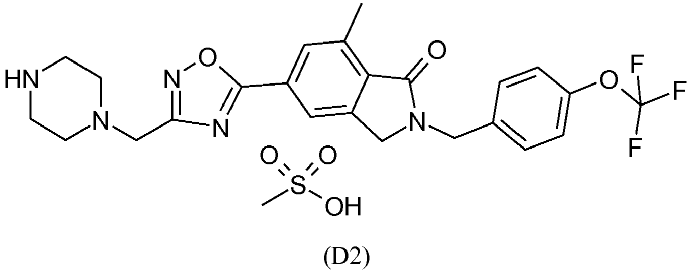

The title compound may be prepared according to Scheme D, as follows. To a solution of 4- 5-[7-methyl-1-oxo-2-(4-trifluoromethoxy-benzyl)-2,3-dihydro-1H-isoindol-5-yl]-[1,2,4]oxadiazol-3-ylmethyl}-piperazine-1-carboxylic acid tert-butyl ester (D1a) (0.170 mol) in a mixture of 1-butanol (250 mL) and water (30 mL) at 85 °C may be added methanesulfonic acid (0.187 mol). After addition of further water (10 mL), the reaction mixture may be held at 85 °C until reaction is complete. The reaction mixture may be cooled to 65-70 °C before conducting a screening filtration into a crystallizer. The reaction vessel and the line can be rinsed into the crystalliser with hot (80 °C) 1-butanol (1 x 200 mL). The resulting reaction mixture may be then held at 85 °C before addition of 1-butanol (800 mL), cooling to 78 °C and seeding with (D2) (0.1 g, 1% w/w). The reaction mixture can then be cooled to 15 °C and temperature-cycled to 65-70 °C twice, before filtration to afford the title compound (D2) which may be washed with 1-butanol (2 x 200 mL) before drying under vacuum to afford the title compound as a white solid (87.38 g, 88.0% yield).
¹H NMR (400MHz, DMSO) 8: 2.36 (3 H, s), 2.81 (4 H, m), 3.15 (4 H, m), 3.91 (2 H, s), 4.46 (2 H, s), 4.78 (2 H, s), 7.37 (2 H, m), 7.45 (2 H, m), 8.00 (1 H, m), 8.12 (1 H, m) and 8.59 (1H, br s).

### REFERENCES

1. Safdar Hayat et al: "An improved method for the synthesis of [gamma]- lactones using sodium bromate and sodium hydrogen sulfite". Tetrahedron Letters, Vol. 42, No.9, 1 February 2001 (2001-02-01), pages 1647-1649.

## Claims

1. A process for preparing 7-methyl-5-(3-piperazin-1-ylmethyl-[1,2,4]oxadiazol-5-yl)-2-(4-trifluoromethoxybenzyl)-2,3-dihydroisoindol-1-one, having the structure below: 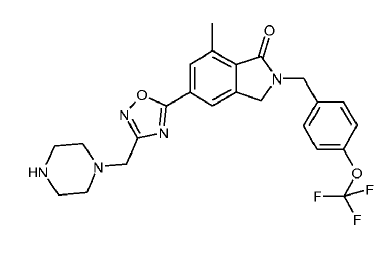 or an acid salt thereof, the process comprising:
reacting tert-butyl piperazine-1-carboxylate with bromoacetonitrile, in an organic solvent in the presence of a base and subsequent reaction with hydroxylamine or an acid salt thereof to form 4-(N-hydroxycarbamimidoylmethyl)-piperazine-1-carboxylic acid tert-butyl ester;
reacting 5-bromo-2-iodo-1,3-dimethyl-benzene in an ether solvent with a Grignard reagent and carbon dioxide gas to form 4-bromo-2,6-dimethylbenzoic acid;
heating 4-bromo-2,6-dimethylbenzoic acid with N-bromosuccinimide and a radical initiator in an organic solvent and subsequent reduction of over-brominated products with a reducing agent to form 5-bromo-7-methyl-3H-isobenzofuran-1-one;
heating 5-bromo-7-methyl-3H-isobenzofuran-1-one with 4-(trifluoromethoxy)-benzylamine in an anhydrous solvent with trimethylaluminium to form 4-bromo-2-hydroxymethyl-6-methyl-N-(4-trifluoromethoxy-benzyl)-benzamide;
reacting 4-bromo-2-hydroxymethyl-6-methyl-N-(4-trifluoromethoxy-benzyl)-benzamide with methanesulphonyl chloride and a base in an organic solvent to form 5-bromo-7-methyl-2-(4-trifluoromethoxy-benzyl)-2,3-dihydro-isoindol-1-one;
heating 5-bromo-7-methyl-2-(4-trifluoromethoxy-benzyl)-2,3-dihydro-isoindol-1-one in the presence of zinc cyanide, 1, 1 '-bis(diphenylphospino)ferrocene and
tris(dibenzylideneacetone)-dipalladium in an organic solvent to form 7-methyl-1-oxo-2-(4-trifluoromethoxy-benzyl)-2,3-dihydro-1H-isoindole-5-carbonitrile;
hydrolysis of 7-methyl-1-oxo-2-(4-trifluoromethoxy-benzyl)-2,3-dihydro-1H-isoindole-5-carbonitrile in a solvent with a hydroxide solution to form 7-methyl-1-oxo-2-(4-trifluoromethoxy-benzyl)-2,3-dihydro-1 H-isoindole-5-carboxylic acid;
reacting 7-methyl-1-oxo-2-(4-trifluoromethoxy-benzyl)-2,3-dihydro-1H-isoindole-5-carboxylic acid in an organic solvent with thionyl chloride or another chlorinating agent and reacting the product with tert-butyl 4-[(2Z)-2-amino-2-hydroxyimino)ethyl]-piperazine-1-carboxylate in the presence of a base to form 4- {5-[7-methyl-1-oxo-2-(4-trifluoromethoxy-benzyl)-2,3-dihydro-1H-isoindol-5-yl]-[1,2,4]oxadiazol-3-ylmethyl}-piperazine-1-carboxylic acid tert-butyl ester, and
decarboxylating 4- {5-[7-methyl-1-oxo-2-(4-trifluoromethoxy-benzyl)-2,3-dihydro-1H-isoindol- 5-yl]-[1,2,4]oxadiazol-3-ylmethyl}-piperazine-1-carboxylic acid tert-butyl ester by heating with acid in a solvent, to form 7-methyl-5-(3-piperazin-1-ylmethyl-[1,2,4]oxadiazol-5-yl)-2-(4-trifluoromethoxy-benzyl)-2,3-dihydro-isoindol-1-one or an acid salt thereof.

2. A compound selected from the group consisting of:
5-bromo-7-methyl-3H-isobenzofuran-1-one;
4-bromo-2-hydroxymethyl-6-methyl-N-(4-trifluoromethoxy-benzyl)-benzamide;

3. A process for making 5-bromo-7-methyl-3H-isobenzofuran-1-one, 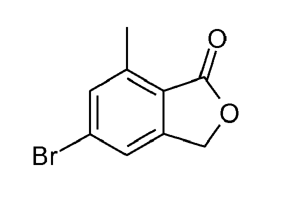 the process comprising:
reacting 5-bromo-2-iodo-1,3-dimethyl-benzene in an ether solvent with a Grignard reagent and carbon dioxide gas to form 4-bromo-2,6-dimethylbenzoic acid, and
heating 4-bromo-2,6-dimethylbenzoic acid with N-bromosuccinimide and a radical initiator in an organic solvent and subsequent reduction of over-brominated products with a reducing agent to form 5-bromo-7-methyl-3H-isobenzofuran-1-one.

4. A process for making 4-bromo-2-hydroxymethyl-6-methyl-N-(4- trifluoromethoxybenzyl)-benzamide, 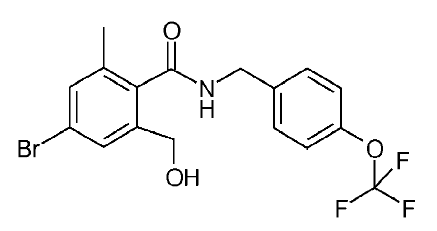 the process comprising:
reacting 5-bromo-2-iodo-1,3-dimethyl-benzene in an ether solvent with a Grignard reagent and carbon dioxide gas to form 4-bromo-2,6-dimethylbenzoic acid;
heating 4-bromo-2,6-dimethylbenzoic acid with N-bromosuccinimide and a radical initiator in an organic solvent and subsequent reduction of over-brominated products with a reducing agent to form 5-bromo-7-methyl-3H-isobenzofuran-1-one;
heating 5-bromo-7-methyl-3H-isobenzofuran-1-one with 4-(trifluoromethoxy)-benzylamine in an anhydrous solvent with trimethylaluminium to form 4- bromo-2-hydroxymethyl-6-methyl-N-(4-trifluoromethoxy-benzyl)-benzamide.

## Patentansprüche

1. Verfahren zur Herstellung von 7-Methyl-5-(3-piperazin-1-ylmethyl[1,2,4]oxadiazol-5-yl)-2-(4-trifluormethoxybenzyl)-2,3-dihydroisoindol-1-on mit der nachstehenden Struktur: 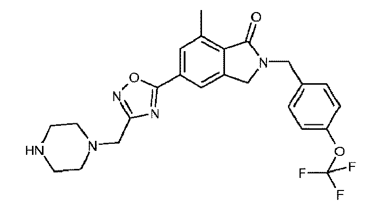 oder ein Säuresalz davon, bei dem man:
Piperazin-1-carbonsäure-tert.-butylester in einem organischen Lösungsmittel in Gegenwart einer Base mit Bromacetonitril und anschließend mit Hydroxylamin oder einem Säuresalz davon zu 4-(N-Hydroxycarbamimidoylmethyl)piperazin-1-carbonsäure-tert.-butylester umsetzt;
5-Brom-2-iod-1,3-dimethylbenzol in einem Ether-Lösungsmittel mit einem Grignard-Reagens und Kohlendioxidgas zu 4-Brom-2,6-dimethylbenzoesäure umsetzt;
4-Brom-2,6-dimethylbenzoesäure in einem organischen Lösungsmittel mit N-Bromsuccinimid und einem Radikalinitiator erhitzt und anschließend überbromierte Produkte mit einem Reduktionsmittel reduziert, wobei man 5-Brom-7-methyl-3H-isobenzofuran-1-on erhält;
5-Brom-7-methyl-3H-isobenzofuran-1-on in einem wasserfreien Lösungsmittel mit Trimethylaluminium mit 4-(Trifluormethoxy)benzylamin erhitzt, wobei man 4-Brom-2-hydroxymethyl-6-methyl-N-(4-trifluormethoxybenzyl)benzamid erhält;
4-Brom-2-hydroxymethyl-6-methyl-N-(4-trifluormethoxybenzyl)benzamid in einem organischen Lösungsmittel mit Methansulfonylchlorid und einer Base zu 5-Brom-7-methyl-2-(4-trifluormethoxybenzyl)-2,3-dihydroisoindol-1-on umsetzt;
5-Brom-7-methyl-2-(4-trifluormethoxybenzyl)-2,3-dihydroisoindol-1-on in Gegenwart von Zinkcyanid, 1,1'-Bis(diphenylphosphino)ferrocen und Tris(dibenzylidenaceton)dipalladium in einem organischen Lösungsmittel erhitzt, wobei man 7-Methyl-1-oxo-2-(4-trifluormethoxybenzyl)-2,3-dihydro-1H-isoindol-5-carbonitril erhält;
7-Methyl-1-oxo-2-(4-trifluormethoxybenzyl)-2,3-dihydro-1H-isoindol-5-carbonitril in einem Lösungsmittel mit einer Hydroxidlösung zu 7-Methyl-1-oxo-2-(4-trifluormethoxybenzyl)-2,3-dihydro-1H-isoindol-5-carbonsäure hydrolysiert;
7-Methyl-1-oxo-2-(4-trifluormethoxybenzyl)-2,3-dihydro-1H-isoindol-5-carbonsäure in einem organischen Lösungsmittel mit Thionylchlorid oder einem anderen Chlorierungsmittel umsetzt und das Produkt in Gegenwart einer Base mit 4-[(2Z)-2-Amino-2-hydroxyimino)ethyl]piperazin-1-carbonsäure-tert.-butylester zu 4-{5-[7-Methyl-1-oxo-2-(4-trifluormethoxybenzyl)-2,3-dihydro-1H-isoindol-5-yl]-[1,2,4]oxadiazol-3-ylmethyl}piperazin-1-carbonsäure-tert.-butylester umsetzt und
4-{5-[7-Methyl-1-oxo-2-(4-trifluormethoxybenzyl)-2,3-dihydro-1H-isoindol-5-yl][1,2,4]oxadiazol-3-ylmethyl}piperazin-1-carbonsäure-tert.-butylester durch Erhitzen mit Säure in einem Lösungsmittel zu 7-Methyl-5-(3-piperazin-1-ylmethyl-[1,2,4]oxadiazol-5-yl)-2-(4-trifluormethoxybenzyl)-2,3-dihydroisoindol-1-on oder einem Säuresalz davon decarboxyliert.

2. Verbindung, ausgewählt aus der Gruppe bestehend aus:
5-Brom-7-methyl-3H-isobenzofuran-1-on;
4-Brom-2-hydroxymethyl-6-methyl-N-(4-trifluormethoxybenzyl)benzamid.

3. Verfahren zur Herstellung von 5-Brom-7-methyl-3H-isobenzofuran-1-on, 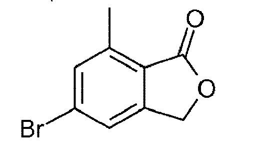 bei dem man:
5-Brom-2-iod-1,3-dimethylbenzol in einem Ether-Lösungsmittel mit einem Grignard-Reagens und Kohlendioxidgas zu 4-Brom-2,6-dimethylbenzoesäure umsetzt; und
4-Brom-2,6-dimethylbenzoesäure in einem organischen Lösungsmittel mit N-Bromsuccinimid und einem Radikalinitiator erhitzt und anschließend überbromierte Produkte mit einem Reduktionsmittel reduziert, wobei man 5-Brom-7-methyl-3H-isobenzofuran-1-on erhält.

4. Verfahren zur Herstellung von 4-Brom-2-hydroxymethyl-6-methyl-N-(4-trifluormethoxybenzyl)benzamid, 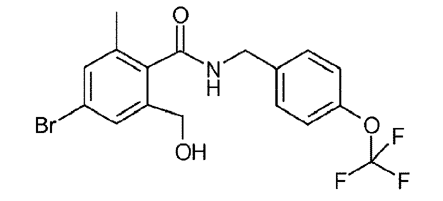 bei dem man:
5-Brom-2-iod-1,3-dimethylbenzol in einem Ether-Lösungsmittel mit einem Grignard-Reagens und Kohlendioxidgas zu 4-Brom-2,6-dimethylbenzoesäure umsetzt;
4-Brom-2,6-dimethylbenzoesäure in einem organischen Lösungsmittel mit N-Bromsuccinimid und einem Radikalinitiator erhitzt und anschließend überbromierte Produkte mit einem Reduktionsmittel reduziert, wobei man 5-Brom-7-methyl-3H-isobenzofuran-1-on erhält;
5-Brom-7-methyl-3H-isobenzofuran-1-on in einem wasserfreien Lösungsmittel mit Trimethylaluminium mit 4-(Trifluormethoxy)benzylamin erhitzt, wobei man 4-Brom-2-hydroxymethyl-6-methyl-N-(4-trifluormethoxybenzyl)benzamid erhält.

## Revendications

1. Procédé de préparation de la 7-méthyl-5-(3-pipérazin-1-ylméthyl-[1,2,4]oxadiazol-5-yl)-2-(4-trifluorométhoxybenzyl)-2,3-dihydroisoindol-1-one, ayant la structure ci-dessous : 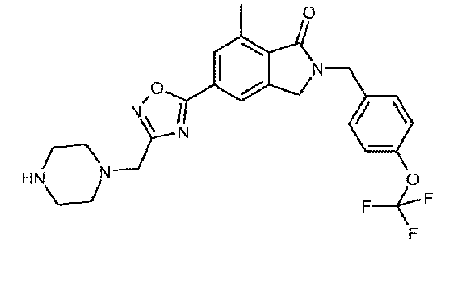 ou un sel d'acide de celle-ci, le procédé comprenant :
la réaction de pipérazine-1-carboxylate de tert-butyle avec du bromoacétonitrile, dans un solvant organique en présence d'une base et ensuite, la réaction avec de l'hydroxylamine ou un sel d'acide de celle-ci pour former l'ester tert-butylique d'acide 4-(N-hydroxycarbamimidoylméthyl)-pipérazine-1-carboxylique ;
la réaction du 5-bromo-2-iodo-1,3-diméthyl-benzène dans un solvant éther avec un réactif de Grignard et du gaz de dioxyde de carbone pour former l'acide 4-bromo-2,6-diméthylbenzoïque ;
le chauffage de l'acide 4-bromo-2,6-diméthylbenzoïque avec du N-bromosuccinimide et un initiateur de radical dans un solvant organique et ensuite, la réduction des produits surbromés avec un agent réducteur pour former la 5-bromo-7-méthyl-3H-isobenzofuran-1-one ;
le chauffage de la 5-bromo-7-méthyl-3H-isobenzofuran-1-one avec de la 4-(trifluorométhoxy)-benzylamine dans un solvant anhydre avec du triméthylaluminium pour former le 4-bromo-2-hydroxyméthyl-6-méthyl-N-(4-trifluorométhoxy-benzyl)-benzamide ;
la réaction du 4-bromo-2-hydroxyméthyl-6-méthyl-N-(4-trifluorométhoxy-benzyl)-benzamide avec du chlorure de méthanesulfonyle et une base dans un solvant organique pour former la 5-bromo-7-méthyl-2-(4-trifluorométhoxybenzyl)-2,3-dihydro-isoindol-1-one ;
le chauffage de la 5-bromo-7-méthyl-2-(4-trifluorométhoxy-benzyl)-2,3-dihydro-isoindol-l-one en présence de cyanure de zinc, de 1,1'-bis(diphénylphosphino)ferrocène et de tris(dibenzylidène-acétone)-dipalladium dans un solvant organique pour former le 7-méthyl-1-oxo-2-(4-trifluorométhoxy-benzyl)-2,3-dihydro-1H-isoindole-5-carbonitrile ;
l'hydrolyse du 7-méthyl-1-oxo-2-(4-trifluorométhoxybenzyl)-2,3-dihydro-1H-isoindole-5-carbonitrile dans un solvant avec une solution d'hydroxyde pour former l'acide 7-méthyl-1-oxo-2-(4-trifluorométhoxy-benzyl)-2,3-dihydro-1H-isoindole-5-carboxylique ;
la réaction de l'acide 7-méthyl-1-oxo-2-(4-trifluorométhoxy-benzyl)-2,3-dihydro-1H-isoindole-5-carboxylique dans un solvant organique avec du chlorure de thionyle ou un autre agent de chloration et la réaction du produit avec du 4-[(2Z)-2-amino-2-hydroxyimino)éthyl]-pipérazine-1-carboxylate de tert-butyle en présence d'une base pour former l'ester tert-butylique d'acide 4-{5-[7-méthyl-1-oxo-2-(4-trifluorométhoxy-benzyl)-2,3-dihydro-1H-isoindol-5-yl]-[1,2,4]oxadiazol-3-ylméthyl}-pipérazine-1-carboxylique, et
la décarboxylation de l'ester tert-butylique d'acide 4-{5-[7-méthyl-1-oxo-2-(4-trifluorométhoxy-benzyl)-2,3-dihydro-1H-isoindol-5-yl]-[1,2,4]oxadiazol-3-ylméthyl}-pipérazine-1-carboxylique par chauffage avec un acide dans un solvant, pour former la 7-méthyl-5-(3-pipérazin-1-ylméthyl-[1,2,4]oxadiazol-5-yl)-2-(4-trifluorométhoxy-benzyl)-2,3-dihydro-isoindol-1-one ou un sel d'acide de celle-ci.

2. Composé choisi dans le groupe constitué des :
5 -bromo-7-méthyl-3H-isobenzofuran-1-one ; 4-bromo-2-hydroxyméthyl-6-méthyl-N-(4-trifluorométhoxybenzyl)-benzamide.

3. Procédé de fabrication de la 5-bromo-7-méthyl-3H-isobenzofuran-1-one, 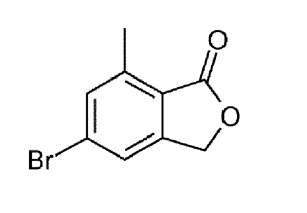 le procédé comprenant :
la réaction de 5-bromo-2-iodo-1,3-diméthyl-benzène dans un solvant éther avec un réactif de Grignard et du gaz de dioxyde de carbone pour former l'acide 4-bromo-2,6-diméthylbenzoïque, et le chauffage d'acide 4-bromo-2,6-diméthylbenzoïque avec du N-bromosuccinimide et un initiateur de radical dans un solvant organique et ensuite, la réduction des produits surbromés avec un agent réducteur pour former la 5-bromo-7-méthyl-3H-isobenzofuran-1-one.

4. Procédé de fabrication du 4-bromo-2-hydroxyméthyl-6-méthyl-N-(4- trifluorométhoxy-benzyl)-benzamide, 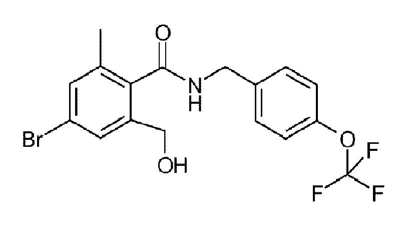 le procédé comprenant :
la réaction de 5-bromo-2-iodo-1,3-diméthyl-benzène dans un solvant éther avec un réactif de Grignard et du gaz de dioxyde de carbone pour former l'acide 4-bromo-2,6-diméthylbenzoïque ;
le chauffage de l'acide 4-bromo-2,6-diméthylbenzoïque avec du N-bromosuccinimide et un initiateur de radical dans un solvant organique et ensuite, la réduction des produits surbromés avec un agent réducteur pour former la 5-bromo-7-méthyl-3H-isobenzofuran-1-one ;
le chauffage de la 5-bromo-7-méthyl-3H-isobenzofuran-1-one avec de la 4-(trifluorométhoxy)-benzylamine dans un solvant anhydre avec du triméthylaluminium pour former le 4-bromo-2-hydroxyméthyl-6-méthyl-N-(4-trifluorométhoxy-benzyl)-benzamide.
